# EUROPEAN PATENT APPLICATION

(11) **EP 1 266 659 A1**
(43) Date of publication of application: **18.12.2002**
(21) Application number: 01202230.7
(22) Date of filing: 11.06.2001
(51) Int. Cl.: A61K 31/44, A61K 31/135, A61K 31/495, A61P 5/00

(54) **Pyridoxal in combination with serotonin re-uptake inhibitor for the treatment of hot flushes**

(71) Applicant: Pantarhei Bioscience B.V., 3700 AL Zeist (NL)
(72) Inventor: Coelingh Bennink, Herman Jan Tymen, 3971 BE Driebergen (NL); van der Linden, René Frank, 2951 LB Maarn (NL)
(74) Representative: Jorritsma, Ruurd

(57) **Abstract**

The present invention is concerned with formulations for use in a method of suppressing hot flushes, especially hot flushes in hypo-estrogenic females and androgen-deprived males. More particularly the invention relates to a pharmaceutical formulation for use in a method of suppressing hot flushes, said method comprising the administration of the formulation so as to provide on a daily basis a combination of:
a. serotonin re-uptake inhibitor in an amount which is equivalent to less than 100 mg trazodone and
b. vitamin B6 component,
in an amount effective to to reduce the incidence and/or intensity of hot flushes

Another aspect of the invention relates to pharmaceutical formulations comprising a combination of
a. serotonin re-uptake inhibitor in an amount which is equivalent to 0.6 to 45 mg, preferably 0.6 to 24 mg trazodone and
b. 0.005 to 5 mmoles of vitamin B6 component,
and additionally comprising pharmaceutically acceptable excipient.

## Description

### FIELD OF THE INVENTION

The present invention is concerned with a method of treating hot flushes, particularly hot flushes in hypo-estrogenic females, such as (peri-)menopausal and post-menopausal females, and also in androgen-deprived males. More particularly the invention relates to methods of suppressing hot flushes by administering a combination of serotonin re-uptake inhibitor and vitamin B6 component in an amount effective to reduce the incidence and/or intensity of hot flushes.

The invention also relates to pharmaceutical formulations comprising a combination of serotonin re-uptake inhibitor and vitamin B6 component.

### BACKGROUND OF THE INVENTION

Serotonin (5-hydroxytryptamine) is a neurotransmitter that is found in relatively high concentrations in the lateral gray horns of the spinal cord and in a number of areas in the brain. A system of serotonin-containing neurons that have their cell bodies in the raphe nuclei of the brain stems project to portions of the hypothalamus, the limbic system, the neocortex and the spinal cord. It has been demonstrated that serotonin interacts with a great number of receptors in the brain and controls or affects processes which regulate many bodily organs and functions.

Serotonin is an important chemical messenger participating in the transmission of nerve impulses in the brain. These messengers are released at specific sites on pre-synaptic cells and received, to complete transmission of the impulse, at specific sites on post-synaptic cells. Their effect is then terminated by metabolism or by uptake into the pre-synaptic cells. In addition, some of the released serotonin is inactivated by monoamine oxidase to form 5-hydroxyindoleacetic acid. This is the principal urinary metabolite of serotonin.

Serotonin is formed in the body by hydroxylation and decarboxylation of the essential amino acid L-tryptophan. In the biosynthesis of serotonin from L-tryptophan, L-tryptophan is hydroxylated in the presence of the enzyme tryptophan hydroxylase to form the intermediate product L-5-hydroxytryptophan. This intermediate product is decarboxylated in the presence of the enzyme 5-hydroxytryptophan decarboxylase to from serotonin.

Serotonin deficiencies in the brain have been associated with a number of mainly psychological disorders such as (endogenous) depression, insomnia, excessive appetite and lowered pain threshold. Recent publications have shown that hot flushes in (peri)menopausal and post-menopausal females may also be related to reduced levels of serotonin.

An important discovery in medicinal chemistry of the past decades are the serotonin re-uptake inhibitors (SRI's), which are particularly effective in the treatment of depression. SRI's increase the availability of serotonin in the synapse by reducing the re-uptake of serotonin by the serotonin uptake carrier (transporter protein). Dysfunction of the serotonin neurons resulting from excessive uptake results in depression, as well as other pathologies of the central nervous system.

Among the commercially available SRI's are fluoxetine, nefazodone, sertraline, venlafaxine, citalopram, fluvoxamine, paroxetine and trazodone. While the primary activity of these drugs is the inhibition of the re-uptake of serotonin, the cascade of monoamine processes in the brain connects serotonin with both norepinephrine and dopamine. Thus, the increase of availability of serotonin may result in increased availability of norepinephrine and dopamine as well.

SRI's have also found application in methods for suppressing hot flushes in methods of decreasing hot flushes in females. EP-A 0 943 329 (Eli Lilly and Company) describes a method for decreasing hot flushes in a human female by administering fluoxetine to that female. An effective dose of fluoxetine is said to range from about 0.001 mg/kg to about 5 mg/kg body weight, per day. The total dosage (per day) of fluoxetine is usually in the range of about 5 mg to 80 mg per day.

Since the commercial introduction of the first SRI's in the early eighties these pharamaceutical components have found widespread application, particularly as antidepressants. Despite this success it is well recognised that, due to a number of undesirable side-effects, such SRI's are to be applied with caution. Examples of side-effects that have reported in connection with the use of SRI's are: effects on sexual function, gastrointestinal symptoms such as nausea, vomiting and diarrhoea, headache, nervousness, insomnia and somnolence (Van den Berg, "Comparing SSRI's; from chemistry to clinical choice", Human Psychopharmacol. (1995), 10, 199-209 and McManus et al., "Nausea and vomiting associated with selective serotonin re-uptake inhibitors - incidence, mechanisms and management", CNS Drugs (1997), 8, 394-401).

In view of the aforementioned side-effects there still is a need for pharmaceutical formulations which deliver the same functionality as existing SRI's, but with less pronounced side effects. It is an objective of the present invention to make available pharmaceutical formulations that comprise one or more SRI's in combination with vitamin B6 component (e.g. vitamin B6) in an amount which effectively enhances the functionality of said one or more SRI's, thus allowing similar results (in terms of suppression of hot flushes) to be achieved at a significantly lower dosage.

US 4,596,807 (Crosby) describes a method for controlling pain, depression and sedation, which method comprises administering a composition comprising a serotonin precursor, such as L-tryptophan or L-5-hydroxytryptophan in an amount effective to increase the brain serotonin to a supra normal level, in combination with a SRI in an amount effective to inhibit the re-uptake of serotonin. The composition may additionally comprise vitamin B6 (pyridoxine) and vitamin C (ascorbic acid) so as to aid biosynthesis of serotonin in case of patients suffering from vitamin depletion. In accordance with this patent, administration of the composition is desirably effected in 1-4 portions daily, delivering 100-800 mg per day of SRI and 40-400 mg per day of the pyridoxine.

US 5,885,976 (Sandyk) is concerned with methods for the treatment of neurological and mental disorders related to deficient serotonin neurotransmission and impaired pineal melatonin functions. The method described comprises administering an effective amount of a composition which increases serotonin transmission followed by the application to the brain of a sufficient amount of an AC pulsed magnetic field to treat the disorder. It is mentioned that the composition may contain a stimulant of serotonin synthesis which is vitamin B1, vitamin B3, vitamin B6, biotin, S-adenosyl methionine, vitamin D, folic acid, ascorbic acid, magnesium, coenzyme Q10, piracetam, or mixtures of two or more thereof. Furthermore it is stated that the composition can include a SRI which is sertraline, nefazodone, trazodone, fluoxetine or a mixture thereof. The ranges of daily dosage levels specifically mentioned for a number of SRI's in this patent broadly cover the range of 25-600 mg.

US 5,254,572 (Serfontein) relates to a method of treatment or prophylaxis of depressed or inadequate intracellular pyridoxal phosphate levels in a patient resulting from a condition, wherein the pyridoxine-pyridoxal phosphate pathway is disturbed or insufficient. The deficiencies are counteracted by the administration of pyridoxal or a precursor of pyridoxal which *in vivo* is rapidly converted into pyridoxal. The methods described are said to be particularly suitable for the treatment or prophylaxis of the broad category of physiological shock, including myocardial infarction.

### SUMMARY OF THE INVENTION

The present invention provides formulations and methods for suppressing hot flushes (also sometimes referred to as hot flashes). The pharmaceutical formulations according to the invention comprise one or more serotonin uptake inhibitors (SRI's) as well as a vitamin B6 component that is capable of enhancing the suppressing action of said one or more SRI's, i.e. acting as a potentiating agent. As a result of the presence of said vitamin B6 component, the levels of SRI needed to achieve the desired suppression can be reduced significantly. Thus the formulations and methods according to the present invention are characterised by the application of relatively low doses of SRI.

Applicants have surprisingly found that vitamin B6 components can advantageously be used in combination with SRI's to increase the effectiveness of these components in suppressing hot flushes. Thus vitamin B6 effectively potentiates the effectiveness of the SRI's against hot flushes. Hence vitamin B6 components are referred to in this document as potentiating agents. The effectiveness of the present method is best demonstrated by the low concentration levels of SRI required to obtain effective suppression of hot flushes. Whereas EP-A 0 943 329 (Eli Lilly) mentions the use of fluoxetine against hot flushes in a concentration range of 1-200 mg per day, and more specifically in the range of 15-40 mg per day, the compositions according to the invention are effective when they contain fluoxetine in quantities that are in the range of 0.1-15 mg per day.

Although the combined use of SRI's and vitamin B6, in areas other than the treatment of hot flushes, has been described in the prior art, it has not been recognised before that, in case of treatment of hot flushes, vitamin B6 has the capability of enhancing the action of SRI's in a way which allows application of SRI's at lower than usual dosage levels. The prior art publications which describe the combined use of SRI's and vitamin B6 teach to employ SRI's at conventional dosage levels. No suggestions are made in these publications that vitamin B6 may act as a potentiating agent for SRI's.

### DETAILED DESCRIPTION OF THE INVENTION

In one embodiment the present invention is concerned with a pharmaceutical formulation comprising a combination of
a. serotonin re-uptake inhibitor in an amount which is equivalent to 0.6 to 45 mg, preferably 0.6 to 24 mg trazodone, more preferably 1 to 20 mg trazodone,
b. 0.005 to 5 mmoles of vitamin B6 component,
and additionally comprising pharmaceutically acceptable excipient.

The term "serotonin re-uptake inhibitor", also sometimes referred to as "serotonin specific (or selective) re-uptake inhibitor (or SSRI)" encompasses those re-uptake inhibitors that are capable of significantly inhibiting re-uptake of serotonin by blocking its transporter protein, by inhibiting monoamine oxidase and/or by (selectively) blocking cerebral serotonin receptors.

The term "vitamin B6 components" as used throughout this document encompasses any components which *in vivo,* particularly once these components or their metabolites have entered the bloodstream, are converted into pyridoxal or a pyridoxal salt. Particularly useful are vitamin B6 components that *in vivo* are converted for at least 10 mol% into pyridoxal or a pyridoxal salt within 24 hours after administration.

Inside living human and animal cells, pyridoxal phosphate and pyridoxamine phosphate are the biologically active forms of vitamin B6, acting as a co-enzymes in more than 100 biological reactions. In the form of pyridoxal 5'-phosphate and pyridoxamine 5'-phosphate, vitamin B6 acts as the coenzyme of a series of enzymes, which catalyse transamination, decarboxylation, deamination,desulphydration and the cleavage or synthesis of amino acids. The aminotransferases represent an important link between amino acid, carbohydrate and fatty acid metabolism and the energy-producing citric acid cycle. The decaboxylases convert amino acids to the corresponding biogenic amines, such as histamine, hydroxytyramine, serotonin, γ-aminobutyric acid, ethanolamine and taurine, some of which are substances of high physiological activity [regulation of the blood vessel diameter, neurohormonal actions, essential components of phospholipids and bile acids]. In accord with the general importance these enzymatic reactions take place in virtually all organs most intensively in the liver, heart and brain. (Lit. Vitamin Compendium; Roche, Vitamins and Chemicals Department. F. Hoffmann- La Roche and Co. Ltd, Basle, Switzerland).

In a preferred embodiment of the invention the pharmaceutical formulation is suitable for oral, enteral or topical administration. Most preferably said formulation is for oral administration.

In order to achieve appreciable increases in cerebral serotonin levels, the formulation will usually contain SRI in an amount which is equivalent to at least 1 mg trazodone and vitamin B6 component in an amount of 0.005 to 5 mmoles. More preferably the amount of SRI utilised is equivalent to 4-20 mg trazodone. The most preferred amount of vitamin B6 component is in the range of 0.01 to 2 mmoles. Formulations for oral dosage containing the aforementioned amounts of SRI and vitamin B6 component are particularly suited for at least once daily administration.

Another embodiment of the present invention is related to a pharmaceutical formulation for use in a method of suppressing hot flushes, said method comprising the administration of the formulation so as to provide on a daily basis a combination of:
a. serotonin re-uptake inhibitor in an amount which is equivalent to less than 100 mg trazodone and
b. vitamin B6 component,
in an amount effective to reduce the incidence and/or intensity of hot flushes. The method of the invention preferably does not include the application to the brain of an AC pulsed magnetic field of at least 7.5 picotesia flux density for at least 15 minutes. More preferably the method does not include the application of an AC pulsed magnetic field of at least 7.5 picotesia. Most preferably the method does not include the application of an AC pulsed magnetic field at all.

The term "on a daily basis", when used in connection with a mentioned dosage amount, should not be interpreted restrictedly. For instance, the above mentioned requirement that the administration of the present formulation is to provide, on a daily basis, SRI in an amount of less than 100 mg trazodone, encompasses a protocol wherein trazodone is administered once a week, provided the dosage is less than 700 mg, i.e. such that the average daily dose is less than 100 mg trazodone.

Co-administration of SRI's with vitamin B6 component was found to be very effective against hot flushes in both females and males. Particularly good results are obtained with the present method in hypo-estrogenic females, particularly (peri-)menopausal and post-menopausal females, and in androgen-deprived males. Here by co-administration is meant that on the one hand SRI and on the other the vitamin B6 component are administered during a time frame wherein the respective periods of pharmacological activity overlap. Thus the term includes sequential as well as simultaneous administration. Since SRI and vitamin B6 component may be co-administered in a sequential as well as in a coextensive fashion the term "pharmaceutical formulation" as used throughout this document should be understood to encompass pharmaceutical kits which comprise separate dosage units for the SRI component and the vitamin B6 component.

The benefits of the formulations according to the invention are particularly pronounced when said formulations are used in a method of controlling hot flushes. Hot flushes are not only experienced by (peri-)menopausal and post-menopausal females, but also occur in hypogonadism, after bilateral ovariectomy and occasionally in the premenstrual syndrome (as a result of hypo-estrogenism). Also androgen-deprived males have been reported to suffer from hot flushes. A hot flush is characterised by a sudden sensation of heat or burning which starts in the head and neck area and then passes, often in waves, over the entire body, but particularly marked in the head, neck, upper chest and back. In most cases hot flushes are accompanied by profuse sweating. The exact pathophysiology of the hot flush is still unknown but it appears to be related to an alteration in the set point temperature in the hypothalamus, the area in which thermosensitive neurons or temperature guardian neurons have been found. Hot flushes are experienced in those periods of the female life after puberty when estrogen levels are low. Hormone replacement therapy (HRT) is thus the first choice for treatment of hot flushes. However, this treatment is not always accepted, may cause undesirable side effects, or is contraindicated for a variety of reasons.

The combination of SRI and vitamin B6 component is deemed to be a good alternative to HRT because HRT has a series of disadvantages. Firstly in women with an uterus the recurrence of menses and vaginal bleeding may be unacceptable. Secondly there are many females who, for a variety of reasons, have serious reservations about HRT-treatment. Thirdly HRT may cause side-effects such as nausea, vomiting, breast tension, headache, mood disturbances, fluid retention, bloating, breakthrough vaginal bleeding, liver function disturbances, cholelithiasis, cholestatic icterus, pancreatitis and a 2-3 fold increased risk of thrombosis. Fourthly HRT is contraindicated in women with a history of unexplained vaginal bleeding, endometrial hyperplasia or cancer, and other estrogen dependant tumours such as breast cancer, thrombo phlebitis, thrombo-embolism, serious liver function disturbances, cholestatic icterus, porphyria and a history of icterus, herpes gestationis or otosclerosis during pregnancy or steroid use.

Surprisingly little attention has been given so far to the strong interactions of estrogens with the serotonergic systems and the role that serotonin plays in the occurrence of hot flushes. It is hypothesised that serotonin is strongly involved in the pathogenisis of hot flushes. In menopausal women, treatment of hot flushes with estrogen can be quite effective. It has been found that such an estrogen replacement therapy augments the serotonergic activity and leads to an increased urinary secretion of 5-hydroxyindol acetic acid, the main metabolite of serotonin. Thus it can be hypothesised that hot flushes occur as a result of serotonin deficiency and that SRI's may suitably be used to reduce hot flushes. According to another preferred embodiment of the method of treatment according to the invention said method comprises at least once daily administration of the pharmaceutical formulation. Since SRI's are metabolised relatively slowly, it is possible to apply longer intervals between doses. However, longer intervals will usually lead to an increase in amplitude of the observed fluctuations of SRI blood serum levels. Since a minimum blood serum level needs to be maintained to achieve the desired results, the maximally occurring blood serum level of SRI will have to be increased with incrementing dosage intervals. This is not in line with the prime aim of the present invention which is to reduce SRI dosage levels, which is why the present method preferably comprises at least once daily administration.

The present method of administering SRI to suppress hot flushes offers the advantage that, thanks to the co-administration of a vitamin B6 component, it employs relatively low levels of SRI. Thus in comparison to the known use of SRI against hot flushes the present method exhibits a decreased incidence of side-effects. This may be explained from the fact that vitamin B6 components in the dose ranges proposed have no side-effects. In a preferred embodiment of the invention the method provides on a daily basis a minimum amount of 0.6 mg SRI, calculated as trazodone equivalent. More preferably the minimum amount is 1 mg trazodone equivalent, most preferably 3 mg trazodone equivalent. The preferred maximum amount is equivalent to less than 90 mg trazodone, more preferably less than 80 mg trazodone. Most preferably the maximum daily amount of SRI provided by the method is equivalent to less than 75 mg trazodone.

The present formulations preferably do not contain a narcotic, specifically not a narcotic selected from the group consisting of codeine, oxycodone, propoxyphene, pentazocine, morphine, meperidine, levorphanol, menthadone and mixtures thereof, in an amount effective to produce analgesia.

The present method of treatment preferably comprises administration of SRI in a daily amount which is equivalent to between 0.01 and 1 mg trazodone per kg bodyweight and between 0.0001 and 0.2 mmoles of the vitamin B6 component per kg bodyweight. More preferably the SRI is administered in an amount of between 0.02 and 0.8 mg trazodone equivalent/kg/day and the vitamin B6 component in an amount between 0.0004 and 0.1 mmoles/kg/day.

Vitamin B6 components which can suitably be used in accordance with the present invention are those selected from the group consisting of pyridoxal, pyridoxamine, acetals of pyridoxal, condensation products arising from the reaction of the aldehyde group of pyridoxal with an amine, and addition salts of any of the foregoing members of the group with pharmaceutically acceptable salts. The term "pharmaceutically acceptable salts" includes salts with pharmaceutically acceptable acids of bases, e.g. acids such as sulphuric, hydrochloric, nitric, phosphoric acid, etc. or bases such as alkali or alkaline earth metal hydroxides, ammonium hydroxides, alkyl ammonium hydroxides etc.

Pyridoxine HCl is the pharmaceutically most widely used form of vitamin B6. However, it has a short half-life in blood *in vivo* as it is readily excreted and/or converted into physiologically unavailable compounds. Also pyridoxine must pass through a chain of biochemical reactions before it can enter the cells and finally end up there as required in the form of active vitamin B6, i.e. pyridoxal phosphate or pyridoxamine phosphate. Thus it can be advantageous to use a vitamin B6 component such as pyridoxal or pyridoxal salt. Preferably, however, the vitamin B6 component used in the present formulation and method is pyridoxine, more preferably the hydrochlorate of pyridoxine, i.e. pyridoxine HCl).

In order to obtain a significant beneficial effect from the co-administration of SRI and vitamin B6 component, it is advisable to apply the vitamin B6 component and SRI in a dosage ratio which is in the range of 0.002 to 1 mmole/mg, more preferably within the range of 0.01 to 0.5 mmole/mg trazodone equivalent.

In a preferred embodiment of the present invention the SRI's employed are selected from the group selected of citalopram, fluoxetine, norfluoxetine, fluvoxamine, paroxetine, sertraline, venlafaxine, zimelidine, femoxetine, trazodone, nefazodone, mirtazapine, pharmaceutically acceptable salts of these inhibitors and mixtures thereof. Most preferably the SRI's are selected from the group consisting of citalopram, fluoxetine, fluvoxamine, paroxetine, sertraline, venlafaxine, trazodone, nefazodone, mirtazapine, pharmaceutically acceptable salts of these inhibitors and mixtures thereof. It is noted that the SRI's used in present formulations may be present in the form of a racemic mixture as well as in the form of a stereo-enantiomer.

Throughout this document amounts of SRI's have been defined in terms of trazodone equivalents. In order to facilitate the translation of given amounts of trazodone into the equivalent amounts of another SRI the following table provides the multiplication factors that are to be used:

| | Trazodone conversion factor |
|---|---|
| citalopram | 0.15 |
| fluoxetine | 0.15 |
| fluvoxamine | 0.7 |
| paroxetine | 0.15 |
| sertraline | 0.3 |
| venlafaxine | 0.5 |
| trazodone | 1.0 |
| mirtazapine | 0.2 |
| nefazodone | 1.3 |

With the help of the above table it can be calculated that 100 mg trazodone is equivalent to 30 mg sertraline or 15 mg fluoxetine.

In another preferred embodiment of the present invention the pharmaceutical formulation additionally comprises at least 300 mg, more preferably from 500 to 10,000 mg of a serotonin precursor selected from the group consisting of L-tryptophan, 5-hydroxytryptophan, precursors of these tryptophan substances and mixtures thereof. The inclusion of the latter precursors may assist in alleviating serotonin deficiency since serotonin is biosynthesised from tryptophane through the following metabolic chain: tryptophan > 5-hydroxytryptophan > 5-hydroxytryptamine > serotonin.

Tryptophan is usually not transported in the blood in a free state, but rather bound to or complexed with blood serum albumin. Tryptophan is the only circulating amino acid that is significantly bound to human blood serum albumin. It has been shown that salicylates displace tryptophan from its protein binding site on albumin in blood plasma thereby raising the free, circulating tryptophan concentration in blood. This free or unbound tryptophan is more easily converted to serotonin than the bound form. Hence the present pharmaceutical formulation may advantageously contain as an additional component a salicylate. Preferably the formulation comprises at least 0.05 mmoles of such a salicylate, more preferably from 0.1 to 1.5 mmoles of the salicylate. Here the term "salicylate" includes both the acid and the salt. The salicylate is preferably selected from the group consisting of sodium salicylate, choline salicylate, magnesium salicylate and mixtures thereof.

Yet another component which may additionally be included in the present formulation is a component that acts as a stimulant of the serotonin receptor, such as buspirone. Hence in another preferred embodiment the formulation comprises at least 10 µmoles, preferably from 20 to 300 µmoles of buspirone.

The pharmaceutical formulations for use in the method of the invention can be solid or semi-solid dosage forms such as tablets, capsules, cachets, pellets, pills, powders and granules, as well as fluid dosage forms such as solutions, emulsions, suspensions, ointments, pastes, creams, gels, jellies and foams. In addition to the pharmacologically active components, the formulation according to the invention contains pharmaceutically acceptable excipient, usually in an amount of between 50 and 99.9 wt.%.

Tablets and equivalent solid and semi-solid dosage forms can suitably contain excipients such binders (e.g. hydroxypropylmethyl cellulose, polyvinyl-pyrrolidine, other cellulosic materials and starch), diluents (e.g. lactose and other sugars, starch, dicalcium phosphate and cellulosic materials), disintegrating agents (e.g. starch polymers and cellulosic materials) and lubricating agents (e.g., stearates and talc).

Transdermal delivery systems include patches, gels, tapes and creams, and can contain excipients such as solubilisers, permeation enhancers (e.g. fatty acids, fatty acid esters, fatty alcohols and amino acids), hydrophilic polymers (e.g. polycarbophil and polyvinyl pyrillidine and adhesives and tackifiers (e.g. polyisobutylenes, silicone-based adhesives, acrylates andpolybutene).

Transmucosal delivery systems include patches, tablets, suppositories, pessaries, gels, and creams, and can contain excipients such as solubilizers and enhancers (e.g. propylene glycol, bile salts and amino acids), and other vehicles (e.g. polyethylene glycol, fatty acid esters and derivatives, and hydrophilic polymers such as hydroxypropylmethyl cellulose and hyaluronic acid).

Injectable delivery systems include solutions, suspensions, gels, microspheres and polymeric injectables, and can comprise excipients such as solubility-altering agents (e.g. ethanol, propylene glycol and sucrose) and polymers (e.g. polycaprylactones, and PLGA's). Implantable systems include rods and discs, and can contain excipients such as PLGA and polycapryl lactone.

Other delivery systems that can be used for administering the pharmaceutical composition of the invention include intranasal delivery systems such as sprays and powders, and sublingual delivery systems.

### EXAMPLE

A clinical study is conducted with 12 peri-menopausal women experiencing at least 40-50 hot flushes per week.

The study is designed as a so called double blind, cross-over study. During the study the medication is orally administered once a day. The visual aspects of the medication used is always the same, i.e. these aspects offer no clues as to the nature of the medication. The volunteers are asked to record the number of hot flushes they experience in a dairy (at least 1 entry per day).

During an initial period of 56 days (days 1-56), all 12 participants receive the same medication, i.e. 20 mg Fluoxetine HCl. Subsequently, the twelve women are randomly divided into two groups, i.e. group A with six women and group B with six women.

Group A is administered the combination of 5mg Fluoxetine HCl + 50mg vitamin B6 during 112 days (days 57-168) and further the combination of 5mg Fluoxetine HCl + placebo during another 112 days (days 169-281). Group B is administered the combination of 5mg Fluoxetine HCl + placebo during days 57-168 and the combination of 5mg Fluoxetine HCl + 50mg vitamin B6 during days 169-281.

Results show that during the period of the first 56 days, the number of hot flushes experienced per day, decreases substantially over time, indicating that 20 mg Fluoxetine HCl as such is efficacious in suppressing hot flushes.

For group A the daily number of hot flushes experienced during days 57-168 is of the same order of magnitude as before the change of medication. During days 169-281, the number of hot flushes increases again over time.

For group B, the daily number of hot flushes experienced during days 57-168 increases over time. However, during days 169-281, when vitamin B6 is taken instead of placebo, the number of hot flushes decreases to substantially lower levels.

## Claims

1. A pharmaceutical formulation for use in a method of suppressing hot flushes, said method comprising the administration of the formulation so as to provide on a daily basis a combination of:
a. serotonin re-uptake inhibitor in an amount which is equivalent to less than 100 mg trazodone and
b. vitamin B6 component,
in an amount effective to reduce the incidence and/or intensity of hot flushes.

2. A pharmaceutical formulation according to claim 1, wherein the method comprises administration of the formulation so as to provide on a daily basis serotonin re-uptake inhibitor in an amount equivalent to 0.6-80 mg trazodone.

3. A pharmaceutical formulation according to claim 1 or 2, wherein the method comprises administration of the formulation so as to provide on a daily basis between 0.005 and 5 mmoles of the vitamin B6 component.

4. A pharmaceutical formulation according to any one of claim 1-3 for use in a method of suppressing hot flushes in hypo-estrogenic females or androgen-deprived males.

5. A pharmaceutical formulation according to any one of claims 1-4, wherein the method comprises at least once daily administration of the formulation.

6. A pharmaceutical formulation according to any one of claims 1-5, wherein the method comprises administration of serotonin re-uptake inhibitor in a daily amount which is equivalent to between 0.01 and 1 mg trazodone per kg bodyweight and between 0.0001 and 0.2 mmoles of the vitamin B6 component per kg bodyweight.

7. A pharmaceutical formulation comprising a combination of
a. serotonin re-uptake inhibitor in an amount which is equivalent to 0.6 to 45 mg, preferably 0.6 to 24 mg trazodone and
b. 0.005 to 5 mmoles of vitamin B6 component,
and additionally comprising pharmaceutically acceptable excipient.

8. A pharmaceutical formulation according to claim 7, wherein the formulation comprises serotonin re-uptake inhibitor in an amount which is equivalent to at least 1 mg trazodone and vitamin B6 component in an amount of 0.01 to 2 mmoles.

9. A pharmaceutical formulation according to any one of claims 1-8, wherein the vitamin B6 component is selected from the group consisting of pyridoxal, pyridoxamine, acetals of pyridoxal, condensation products arising from the reaction of the aldehyde group of pyridoxal with an amine, and addition salts of any of the foregoing members of the group with pharmaceutically acceptable salts,

10. A pharmaceutical formulation according to any one of claims 1-9, wherein the dosage ratio of vitamin B6 component to serotonin re-uptake inhibitor is in the range of 0.002 to 1 mmole/mg trazodone equivalent.

11. A pharmaceutical formulation according to any one of claims 1-10, wherein the serotonin re-uptake inhibitor is selected from the group selected of citalopram, fluoxetine, norfluoxetine, fluvoxamine, paroxetine, sertraline, venlafaxine, zimelidine, femoxetine, trazodone, nefazodone, mirtazapine, pharmaceutically acceptable salts of these inhibitors and mixtures thereof.

12. A pharmaceutical formulation according to any one of claims 1 - 11, wherein the formulation comprises at least 300 mg of a serotonin precursor selected from the group consisting of L-tryptophan, 5-hydroxytryptophan, precursors thereof and mixtures thereof.

13. A pharmaceutical formulation according to any one of claims 1-12, wherein the formulation comprises at least 0.05 mmoles of a salicylate.
